# EUROPÄISCHE PATENTSCHRIFT

(11) **EP 2 674 116 B1**
(45) Veröffentlichungstag und Bekanntmachung des Hinweises auf die Patenterteilung: **06.08.2014**
(21) Anmeldenummer: 12171416.6
(22) Anmeldetag: 11.06.2012
(51) Int. Cl.: A61B 17/16

(54) **Werkzeughalte- und Griffteil für ein medizinisches, insbesondere chirurgisches, Werkzeug**
Tool holding and gripping part for a medical tool, in particular a surgical tool
Pièce de saisie et de retenue d'outil pour un outil médical, en particulier chirurgical

(43) Veröffentlichungstag der Anmeldung: 18.12.2013
(73) Patentinhaber: Waldemar Link GmbH & Co. KG, 22339 Hamburg (DE)
(72) Erfinder: Dmuschewsky, Klaus, 22397 Hamburg (DE)
(74) Vertreter: Raffay & Fleck

(56) Entgegenhaltungen:
- US-A1- 2008 255 565
- US-A1- 2010 331 902
- US-B1- 6 663 636

## Beschreibung

Die vorliegende Erfindung betrifft ein Werkzeughalte- und Griffteil zum lösbaren Verbinden mit einem medizinischen, insbesondere chirurgischen, Werkzeug mit den Merkmalen des Oberbegriffs des Anspruches 1.

Es ist bekannt, medizinische Instrumente, insbesondere chirurgische Instrumente, aus lösbar miteinander verbindbaren Elementen eines Griff- und Halteteils sowie des eigentlichen Werkzeugteils zu bilden. Dies ist insbesondere von Vorteil, da auf diese Weise mit einem universellen Griff- und Halteteil zur Bildung unterschiedlicher medizinischer Instrumente verschiedene, beispielsweise verschieden geformte oder aber auch in der Wirkung unterschiedliche Werkzeugteile verbunden werden können.

Dabei müssen die Werkzeuge an dem Werkzeughalte- und Griffteil einerseits schnell und einfach auswechselbar, andererseits aber im Einsatz sicher haltend mit dem Werkzeughalte- und Griffteil verbunden werden. Bekannte Beispiele derartiger Instrumente, bei denen die Werkzeuge als Knochenraspeln beschrieben werden, sind in den Druckschriften DE 195 01 882 C2 und DE 298 07 671 U1 angegeben. Bei der in der erstgenannten Druckschrift offenbarten Lösung findet eine Anbindung und Verriegelung über einen mit einem sternförmigen Ansatz versehenen Haltezapfen an dem Werkzeugteil statt, der in gegenüber der Normalrichtung verdrehter Ausrichtung mit den Sternstrahlen zwischen entsprechenden Vorsprüngen eines Aufnahmekanals in dem Werkzeughalte- und Griffteil an diesem vorbei geführt und anschließend um einen bestimmten Winkelbetrag verdreht wird in die Gebrauchsstellung. In dieser Stellung greifen die Vorsprünge in dem Haltekanal hinter die sternförmigen Fortsätze am Ende des Haltedorns. Als zusätzliche Verriegelung fährt ein federbelasteter Riegelbolzen in eine entsprechende Ausnehmung an dem Werkzeug, um ein unbeabsichtigtes Zurückdrehen und Lösen des Werkzeuges zu verhindern.

In dem Beispiel der zweitgenannten Druckschrift findet die Verbindung zwischen dem Werkzeughalte- und Griffteil und dem Werkzeug selbst in einem Verbindungsabschnitt statt, der gegenüber einer Längsrichtung des Werkzeuges wie auch des Halte- und Griffteils gekröpft verläuft. Ein mittels eines Schiebers bewegbarer Riegelstift, der sich entlang der Längsrichtung des Werkzeughalte- und Griffteils erstreckt und bewegen lässt, wird bei schräg zu dessen Längsrichtung verlaufendem und in eine entsprechend schräg gelegene Aufnahme an dem Griffteil eingeschobenem Haltezapfen in eine entsprechende Arretierungsöffnung in diesem Zapfen eingefahren und verriegelt so das Werkzeug.

Ein weiteres aus dem Stand der Technik bekanntes Beispiel eines Werkzeughalte- und Griffteils, von dem die Erfindung hier ausgeht, ist in der US 6,663,636 B1 gezeigt. Dort ist ein Riegelmittel in Form eines an einem Hebelelement angeordneten Fortsatzes vorgesehen, welches Riegelmittel über eine Hebelanordnung zwischen einer Freigabeposition und einer Riegelposition, in welcher das Riegelmittel an einem Rücksprung eines in eine Aufnahmeöffnung an dem Halte- und Griffteil angeführten Verbindungszapfens des Werkzeuges eingreift, und einer Öffnungsposition, in welcher das Riegelmittel den Zapfen freigibt, verschwenkt werden kann. Die Hebelanordnung besteht hierbei aus insgesamt drei Hebelelementen, von denen ein erstes das Riegelmittel trägt, ein zweites eine Verbindungsstrebe zur Übertragung der Hebelkraft darstellt und ein drittes einen Betätigungshebelarm umfasst. Durch Verschwenken des Betätigungshebelarms wird somit eine Kraft auf das das Riegelmittel tragende Hebelelement ausgeübt und dieses zwischen der Riegelposition und der Freigabeposition verschwenkt bzw. bewegt.

Ein weiteres Werkzeughalte- und Griffteil, welches insgesamt die Merkmale des Oberbegriffs des Anspruchs 1 zeigt, ist in der US 2008/0255565 A1 offenbart. Dort sind zwei über miteinander grundsätzlich lösbar und unmittelbar verbundene Lagerabschnitte gekoppelte Hebelelerriente jeweils mit einem durch Bohrungen in dem Grundkorpus und dem jeweiligen Hebelelement geführte Lagerstifte schwenkbar an dem Grundkorpus festgelegt. Die Lagerstifte sind dabei in dem Grundkorpus fest verankert, so dass sich keine Möglichkeit zur einfachen Demontage des dort gezeigten Werkzeughalte- und Griffteils ergibt.

Zwar ist allen vorbekannten Lösungen gleichermaßen gemein, dass sie eine sichere und zuverlässige Anbindung des Werkzeuges an das Werkzeughalte- und Griffteil ergeben und somit eine zuverlässige Nutzung bzw. einen sicheren Einsatz des so gebildeten Instrumentes bedingen. Jedoch sind die vorstehend bezeichneten Lösungen dahingehend verbesserungswürdig, als dass sie für eine nach dem Einsatz erforderliche Reinigung und Sterilisierung gewisse Hindernisse und Schwierigkeiten mit sich bringen. Denn typischerweise verschmutzen bei einem typischen operativen Einsatz eines medizinischen Instrumentes, welches aus dem Werkzeug und dem Werkzeughalte- und Griffteil gebildet ist, nicht nur das Werkzeug selbst, sondern auch das Werkzeughalte- und Griffteil. Diese Verschmutzungen sind dabei insbesondere Blut und Gewebereste, ggf. auch Knochenabrieb oder Verunreinigungen mit anderen Körperflüssigkeiten als Blut. Da es sich bei diesen Gegenständen klassisch um mehrfach verwendbare Instrumente bzw. Teile handelt, müssen diese nach einem Gebrauch entsprechend sorgfältig von den anhaftenden Verunreinigungen gereinigt und sterilisiert werden, um so etwaige Übertragungen von Krankheiten oder durch Einbringen fremder Gewebeteile oder Körperflüssigkeiten in das Körpersystem eines weiteren Patienten hervorgerufene Immunreaktionen zu vermeiden.

Bei derartigen zu unternehmenden Reinigungen sind insbesondere enge Zwischenräume oder Hohlräume schwierig zu behandeln, wie sie bei den aus dem Stand der Technik bekannten Gestaltungsformen gegeben sind. Bei dem aus der DE 195 01 882 C2 bekannten Werkzeughalte- und Griffteil ist dies insbesondere die Sacklochbohrung, in welcher der Riegelstift angeordnet ist und der Schlitz, durch welches das Griffteil dieses Riegelstiftes hindurchragt. Bei dem aus der DE 298 07 671 U1 bekannten Element ist dies die gesamte Führung der Riegelstange. Bei dem aus der US 6,663,636 B1 bekannten Werkzeughalte- und Griffteil sind dies der Aufnahmeraum, in welchem die einzelnen Hebelelemente angeordnet sind und insbesondere die Bereiche zwischen den Hebelelementen und der Wandung des Griffteils. Bei dem Werkzeughalte- und Griffteil nach der US 2008/0255565 A1 sind dies der Raum der Lagerverbindungsabschnitte der beiden Hebelelemente und die angrenzenden Flächen der Hebelelemente und der in dem Griffteil gebildeten Aufnahme. Dabei wirkt sich insbesondere aus, dass die genannten Instrumente in ihren Elementen nicht weiter zerlegbar gestaltet sind, was insbesondere auch auf die in der US 6,663,636 und der US 2008/0255565 A1 gezeigten Werkzeughalte- und Griffteile und dort insbesondere auf die Hebelanordnungen zutrifft. Bei der US 6,663,636 sind die einzelnen Elemente der Hebelanordnung über Lagerstifte miteinander verbunden bzw. an dem Grundkorpus des Werkzeughalte- und Griffteils angelagert, die nicht zum Zerlegen vorgesehen sind. Bei dem Werkzeughalte- und Griffteil der US 2008/0255565 A1 sind zwar die beiden Hebelelemente miteinander nicht über eine Stiftverbindung gekoppelt. Dennoch sind die beiden Hebelelemente an sich über Lagerstifte an dem Grundkorpus festgelegt, wobei diese Verbindungen ebenfalls nicht zum Zerlegen vorgesehen sind. Zwar könnte man theoretische diese Haltestifte herausnehmbar und damit das jeweilige Werkzeughalte- und Griffteil zerlegbar gestatten, jedoch entstünden dabei eine Vielzahl von Kleinteilen, die ein Zerlegen und Zusammensetzen durch das entsprechende medizinische Hilfspersonal erschweren und zudem verlorengehen können. Auch besteht bei derartigen Kleinteilen immer die Gefahr, dass sie sich während eines operativen Einsatzes des Instrumentes lösen und in einer Operationswunde verbleiben.

Hier nun Abhilfe zu schaffen und ein Werkzeughalte- und Griffteil der eingangs genannten Art dahin fortzubilden, dass es bei weiterhin zuverlässiger Haltewirkung auf das Werkzeug eine deutlich vereinfachte Reinigung und Sterilisierung nach dem Gebrauch erlaubt, hierzu insbesondere mit wenigen und einfachen Handgriffe in wenige und einfach zu reinigende Teile ohne Kleinteile wie Verbindungsstifte oder dgl. zerlegbar ist, ist Aufgabe der vorliegenden Erfindung.

Diese Aufgabe wird gelöst durch ein Werkzeughalte- und Griffteil zum lösbaren Verbinden mit einem medizinischen, insbesondere chirurgischen, Werkzeug mit den Merkmalen des Anspruches 1. Vorteilhafte Weiterbildungen eines solchen Werkzeughalte- und Griffteils sind in den abhängigen Ansprüchen 2 bis 13 angegeben. In weiteren Aspekten gibt die Erfindung mit Anspruch 14 ein medizinisches, insbesondere chirurgisches, Instrument an, welches aus einem erfindungsgemäßen Werkzeughalte- und Griffteil sowie einem mit diesem verbundenen medizinischen, insbesondere chirurgischen, Werkzeug gebildet ist. Schließlich ist ein weiterer Aspekt der Erfindung in einem Set zum Bilden eines medizinischen, insbesondere chirurgischen, Instrumentes zu sehen, welches aus einem Werkzeughalte- und Griffteil gemäß dieser Erfindung sowie wenigstens zwei unterschiedlichen medizinischen, insbesondere chirurgischen, Werkzeugen besteht.

Im Sinne dieser Erfindung ist unter einem "medizinischen, insbesondere chirurgischen, Werkzeug" nicht lediglich ein Werkzeug im engeren Sinne, also ein solches zum Bearbeiten körpereigenen Materials, wie Muskelgewebe oder Knochen, oder für die Versorgung eines Patienten vorgesehener Hilfsmittel, wie beispielsweise Implantate, Knochenschrauben oder dgl., zu verstehen. Unter diesen Begriff gefasst sein sollen insbesondere auch medizinische Behandlungsmittel, die mit einem derartigen Halte- und Griffteil verbunden werden können, beispielsweise Implantatbestandteile, die mittels eines solchen Werkzeughalte- und Griffteils gesetzt oder aber entfernt werden können.

Erfindungsgemäß weist ein neuartiges Werkzeughalte- und Griffteil zum lösbaren Verbinden mit einem medizinischen, insbesondere chirurgischen, Werkzeug in Übereinstimmung mit dem Stand der Technik zunächst einen Grundkorpus sowie eine Hebelanordnung aus miteinander verbundenen, jeweils an dem Grundkorpus schwenkbar gelagerten Hebelelementen auf. Der Grundkorpus ist dabei einteilbar in einen Griffabschnitt, der dem Ergreifen und Handhaben des Werkzeughalte- und Griffteils dient, und einen Verbindungsabschnitt, an welchem das Werkzeughalte- und Griffteil mit dem chirurgischen Werkzeug verbunden wird und welcher hierfür typischerweise entsprechende Verbindungsstrukturen bzw. - mittel aufweist. In dem Verbindungsabschnitt ist dazu ein aus einer Freigabeposition in eine Riegelposition bewegbares Riegelmittel vorgesehen, was dem Verriegeln eines an dem Verbindungsabschnitt angesetzten Werkzeuges dient. In der Riegelposition verriegelt das Riegelmittel das Werkzeug entsprechend, in der Freigabeposition wird das Werkzeug zum Lösen von dem Werkzeughalte- und Griffteil freigegeben.

Von den Hebelelementen weist ein erstes einen Betätigungshebelarm auf, ein zweites bewegt das Riegelmittel, welches in bevorzugter Ausgestaltung an einem Hebelarm dieses zweiten Hebelelementes angeformt ist. Ferner ist auch bei dem erfindungsgemäßen Werkzeughalte- und Griffteil die Hebelanordnung aus genau zwei Hebelelementen gebildet, welche jeweils mit daran ausgeformten Schwenklagerabschnitten gegenüber dem Grundkorpus verschwenkbar an in dem Grundkorpus ausgeformten Schwenklagerstrukturen angeordnet sind.

Weiterhin weisen bei dem erfindungsgemäßen Werkzeughalte- und Griffteil die beiden Hebelelemente jeweils an einem ihrer Hebelarme einen Lagerverbindungsabschnitt auf, welche Lagerverbindungsabschnitte bei zusammengefügtem Werkzeughalte- und Griffteil lösbar und unmittelbar miteinander verbunden sind (zum Zusammenfügen des Werkzeughalte- und Griffteils also entsprechend miteinander verbindbar sind) und im Zusammenwirken ein relativ zu dem Grundkorpus verlagerbares Verbindungsschwenklager für die beiden Hebelelemente bilden. Mit einer unmittelbaren Verbindung der beiden Lagerverbindungsabschnitte ist hier solche gemeint, die verbindungsmittelfrei ist, also ohne weitere Elemente, wie etwa in Bohrungen eingeführte Lagerstifte oder dgl. auskommt. Vielmehr werden die beiden Lagerverbindungsabschnitte der beiden Hebelelemente unmittelbar miteinander in Verbindung gebracht und bilden so das Verbindungsschwenklager.

Insoweit stimmt auch das erfindungsgemäße Werkzeughalte- und Griffteil in seinen Merkmalen noch mit demjenigen überein, welches aus der US 2008/0255565 A1 bekannt ist. Im Unterschied zu diesem zeichnet sich das erfindungsgemäße Werkzäughalte- und Griffteil nun dadurch aus, dass die beiden Hebelelemente mit ihren Schwenklagerabschnitten an den in dem Grundkorpus ausgeformten Schwenklagerstrukturen unmittelbar und abnehmbar angeordnet sind.

Die unmittelbare Anordnung der Schwenklagerabschnitte der Hebelelemente an dem Grundkorpus bedeutet dabei wiederum, dass dies verbindungsmittelfrei geschieht, also ohne weitere Verbindungsteile, wie etwa Lagerstifte oder dgl., wie sie in der US 6,663,636 B1 oder auch der US 2008/0255565 A1 eingesetzt werden. Vielmehr sind die an den Hebelelementen gebildeten Schwenklagerabschnitte Bestandteile der Hebelelemente selbst und so geformt, dass sie in entsprechende Schwenklagerstrukturen des Grundkorpus eingesetzt bzw. auf solche aufgesetzt werden können und bereits so die Schwenklagerverbindung bilden. Diese ist dabei auch noch derart gestaltet, dass sich eine lösbare Verbindung ergibt, so dass die einzelnen Hebelelemente einfach von dem Gründkorpus abgenommen und dabei die Schwenklagerverbindungen gelöst werden können.

Insbesondere ist es dabei bevorzugt so, dass bei dem erfindungsgemäßen Werkzeughalte- und Griffteil die Festlegung der beiden Hebelelemente an dem Grundkorpus dadurch erfolgt, dass die zunächst einzeln mit den Schwenklagerabschnitten an den Schwenklagerstrukturen in dem Grundkorpus angeordneten Hebelelemente mit den Lagerverbindungsabschnitten zur Bildung des Verbindungsschwenklagers untereinander verbunden werden und auf diese Weise die Hebelanordnung fixieren. Für ein Zerlegen des erfindungsgemäßen Werkzeughalte- und Griffteils wird bei dieser Lösung in umgekehrter Weise zunächst - typischerweise durch Überwinden einer entsprechenden Haltekraft - dasVerbindungsschwenklager getrennt, werden die beiden Lagerverbindungsabschnitte der einzelnen Hebelelemente voneinander gelöst, und können dann die beiden Hebelelemente einfach mit ihren Schwenklagerabschnitten von den Schwenklagerstrukturen des Grundkorpus gelöst und entnommen werden.

Im Ergebnis wird somit also ein einfaches, im bevorzugten Falle, in dem das Riegelmittel unmittelbar mit dem zweiten Hebelelement verbunden ist, insgesamt nur dreiteiliges und auf einfache Weise in diese drei Teile zerlegbares Werkzeughalte- und Griffteil angegeben, welches nicht nur einfach zerlegt und zusammengefügt werden kann, sondern im zerlegten Zustand vergleichsweise groß dimensionierte Teile ergibt, die mit vergleichsweise einfacher Struktur insbesondere leicht zu reinigen und sterilisieren sind. Die Kraftübertragung auf das Riegelmittel bzw. die Bewegung des Riegelmittels zum Verriegeln eines angesetzten medizinischen, insbesondere chirurgischen Werkzeuges geschieht dabei in ähnlicher und gleichsam zuverlässiger Weise wie beim Stand der Technik gemäß der US 6,663,636 B1 oder der US 2008/0255565 A1.

Dabei ist typischerweise und mit Vorteil in dem Verbindungsabschnitt des Werkzeughalte- und Griffteils eine weitere Struktur zum Ansetzen einer entsprechenden Gegenstruktur an dem Werkzeug vorgesehen. Derzeit bevorzugt ist dieses Paar aus miteinander in Verbindung zu bringenden Strukturen gebildet durch einen Aufsatzzapfen, der in dem Verbindungsabschnitt des Werkzeughalte- und Griffteils mit diesem einstückig gebildet ist und mit einer entsprechenden Zapfenaufnahme an dem Werkzeug zusammenwirkt. Bevorzugt ist dieser Zapfen dabei von einem von der Kreisform abweichenden Durchmesser, um hier eine Verdrehsicherung zu bewirken. Insbesondere weist dieser Zapfen eine - ggf. mit abgerundeten kurzen Seiten versehene - in der Grundform trapezartige Querschnittstruktur auf. Eine solche Struktur hat den Vorteil, dass ein anzusetzendes Werkzeug nur in einer bestimmten Richtung aufgesetzt werden kann, so dass sich hier Fehlbedienungen ausschließen lassen. Das Werkzeug kann dabei auch eine entsprechende Aufnahmestruktur zum Aufnehmen des Riegelmittels aufweisen, mit welchem dieses in der Riegelposition zum sicheren Verriegeln des aufgesetzten Werkzeuges zusammenwirkt.

In einer vorteilhaften Weiterbildung der Erfindung ist vorgesehen, dass der Lagerverbindungsabschnitt an einem der Hebelelemente einen mit seiner Längsrichtung quer zu der Erstreckungsrichtung des Hebelarmes, an dem er angeordnet ist, verlaufenden, an diesem Hebelarm festgelegten, insbesondere einstückig angeformten, Kreiszylinderabschnitt aufweist. In dieser Kombination umfasst der Lagerverbindungsabschnitt an dem anderen der Hebelelemente eine in einem Gabelgrund mit einem zu einem Radius des Kreiszylinderabschnittes korrespondierenden Radius ausgeformte, durch seitliche Schenkel begrenzte Aufnahmegabel. Es ist einfach zu erkennen, dass eine derartige Anordnung durch Zusammenfügen der beiden Lagerverbindungsabschnitte ein entsprechendes Verbindungsschwenklager ergibt, ohne dass es hierfür weiterer Bestandteile zum Verbinden oder als Drehachse bedarf. Die Ausformung mit Kreiszylinderabschnitt und korrespondierender Aufnahmegabel bewirkt dabei zudem eine definierte Reduzierung der Verschwenkbarkeit auf eine solche um eine einzige Achse, nämlich die Längsachse des Kreiszylinderabschnittes. Schwenkbewegungen in andere Richtungen sind bei zusammengefügtem Verbindungsschwenklager durch die die Aufnahmegabel begrenzenden Schenkel unterbunden bzw. blockiert

Gemäß einer weiteren Weiterbildung dieser Lösung sind die Schenkel der Aufnahmegabel ungleich lang, wobei der bei einer Schließbewegung der Hebelanordnung, also einer Bewegung, die diese beim Bewegen des Riegelmittels in Riegelposition ausführt, Kraft übertragende Schenkel der längere ist. Gerade in dieser Bewegungsrichtung muss zum Verriegeln des Werkzeuges zuverlässig eine vergleichsweise hohe Riegelkraft auf das Riegelelement aufgebracht werden, was zu einer entsprechenden Belastung der lösbaren Verbindung an dem Verbindungsschwenklager führt. Auch muss in dieser Bewegungsrichtung ein unbeabsichtigtes Lösen des Verbindungsschwenklagers zuverlässig verhindert werden. Entsprechend ist eine längere, ggf. auch in der Materialstärke stärkere, Ausbildung des die Kraft übertragenden Schenkels von Vorteil.

Ferner kann gemäß einer weiteren vorteilhaften Weiterbildung die durch Aufnahmegabel und Kreiszylinderabschnitt gebildete Ausgestaltung der Lagerverbindungsabschnitte das Verbindungsschwenklager vorgegeben sein, wie in Anspruch 4 bezeichnet. Demnach haben die Schenkel der Aufnahmegabel an ihren freien Enden eine Öffnungsweite, die geringer ist als der Durchmesser des Kreiszylinderabschnittes, und einer der Schenkel ist als Federzunge ausgebildet. Dies ist mit Vorteil derjenige Schenkel, der bei der vorstehend definierten Schließbewegung der Hebelanordnung die Kraft nicht überträgt, sondern entlastet ist. Bei einer Ausbildung mit ungleich langen Schenkeln wie vorstehend beschrieben, kann dies also insbesondere der kürzere Schenkel sein. Durch eine derartige Ausgestaltung der Aufnahmegabel kann der von dem freien Ende der Schenkel her in die Gabel eingeführte Kreiszylinderabschnitt unter Auswärtsverlagerung des als Federzunge ausgebildeten Schenkels in die Gabel eingedrückt werden und wird dann, wenn er mit seinem Durchmesser das äußere freie Ende des als Federzunge ausgebildeten Schenkels passiert hat und dieser Schenkel in seine Ruhestellung zurückspringt, in der Gabel gesichert. Auf diese Art und Weise ist das Verbindungsschwenklager gegen ein unbeabsichtigtes Lösen der zusammenwirkenden Lagerverbindungsabschnitte gesichert, kann bei in den Grundkorpus eingesetzten Hebelelementen und zusammengefügtem Verbindungsschwenklager die Anordnung der Hebelanordnung und deren schwenkbare Verbindung mit dem Grundkorpus gesichert werden.

Bei einer weiteren vorteilhaften Weiterbildung der Erfindung ist es möglich, dass einer der Hebelarme der Hebelelemente, an denen die Lagerverbindungsabschnitte angeordnet sind, insbesondere der mit dem Lagerverbindungsabschnitt versehene Hebelarm des das Riegelelement bewegenden zweiten Hebelelementes, als Federarm ausgebildet sein mit einer Federelastizität in Richtung quer zu seiner Längserstreckung. Auch eine darin ausgebildete Federwirkung kann dabei unterstützen, das Verbindungsschwenklager lösbar, in einer zusammengesetzten Verbindung der Lagerverbindungsabschnitte jedoch gegen eine Federkraft in der Verbindungsstellung gehalten zu gestalten. Mit Vorteil ist der als Federarm ausgebildete Hebelarm in einer der Kraft, die bei der Schließbewegung der Hebelanordnung auf diesen wirkt, entgegengesetzten Richtung gekrümmt.

Mit Vorteil sind insgesamt die in dem Verbindungsschwenklager lösbar verbundenen Schwenklagerabschnitte und die Hebelelemente derart ausgebildet, dass bei einem Überstrecken der Hebelanordnung, insbesondere bei einem Überstrecken im Zuge der Öffnungsbewegung, die die Hebelanordnung beim Bewegen des Riegelmittels in die Freigabeposition ausführt, die Schwenklagerabschnitte durch Überwinden einer Rückhaltekraft voneinander trenn- und lösbar sind. Auf diese Weise ergibt sich ein einfaches Auftrennen dieser Verbindung, wonach die beiden Hebelelemente einfach mit ihren Schwenklagerabschnitten von den Schwenklagerstrukturen des Grundkorpus gelöst und abgenommen werden können. Die Rückhaltekraft ist dabei entsprechend so zu bemessen, dass im normalen Gebrauch ein versehentliches Lösen der Lagerverbindungsabschnitte ausgeschlossen ist, auch nach wiederholtem und mehrfachem Gebrauch. Andererseits darf diese Rückhaltekraft nicht zu groß werden, so dass ein einfaches Trennen dieser Verbindung auch von körperlich weniger robustem medizinischem Personal möglich ist.

Eine Möglichkeit, einen oder beide Hebelelemente an dem Grundkorpus verschwenkbar und lösbar sowie unmittelbar zu lagern, besteht darin, an dem wenigstens einen Hebelelement einen Kreiszylinderabschnitt als Lagerabschnitt auszubilden und als mit diesem zusammenwirkende Schwenklagerstruktur eine Aufnahmekehle in dem Grundkorpus mit einem in einem dem Radius des Kreiszylinderabschnittes entsprechenden Radius gekrümmten Kehlengrund. Diese Aufnahmekehle ist einseitig offen mit einer Öffnungsweite, in die der Kreiszylinderabschnitt eingebracht und so zum Verschwenken auf den Kehlengrund aufgesetzt werden kann.

Eine weitere Möglichkeit einer lösbaren Anlagerung eines Hebelelementes an dem Grundkorpus besteht darin, dass als Lagerabschnitt in dem Hebelelement eine U-förmige Lagerkehle mit einem in einem Radius gekrümmt geführten Kehlengrund gebildet ist und die Schwenklagerstruktur durch einen fest an dem Grundkorpus angeordneten Lagerstift mit zu dem Radius des Kehlengrundes korrespondierendem Radius realisiert ist. Anstelle eines durchgehenden Lagerstiftes kann hier auch ein Kreiszylinderabschnitt gewählt werden, der an dem Grundkorpus geformt ist, wobei die Umfangsweite des Kreiszylinders so gewählt sein muss, dass der Schwenkweg des entsprechenden Hebelelementes gewährleistet werden kann durch einen Lauf des Kehlengrundes der U-förmigen Lagerkehle an dem Hebelelement. Die U-förmige Lagerkehle ist wiederum einseitig offen mit einer Öffnungsweite, die ein einfaches Aufsetzen dieser Lagerkehle auf den Lagerstift bzw. eine entsprechende einen Kreiszylinderabschnitt aufweisende Struktur ermöglicht, ohne dass weiter Befestigungselemente bzw. -mittel erforderlich wären.

Mit Vorteil ist in einem Abschnitt des Grundkorpus ein zumindest teilweise durchgehender, d.h. diesen Grundkorpus zu beiden Seiten durchbrechender Aufnahmeschlitz gebildet, in welchem die Hebelelemente jeweils wenigstens mit Teilen ihrer Strukturen aufgenommen sind und wobei die Schwenklagerstrukturen zum schwenkbaren Lagern der Hebelelemente in dem Aufnahmeschlitz angeordnet sind. Somit ist die Hebelanordnung mit weiten Bereichen im Inneren des Werkzeughalte- und Griffteils angeordnet. Typischerweise wird allerdings zumindest der Betätigungshebelarm aus dem Aufnahmeschlitz herausragen, da dieser von einer Bedienperson, typischerweise einem Arzt oder einem medizinischen Assistenz (OP-Helfer) zu bedienen ist.

Um ein Überschwenken in Schließrichtung der Hebelanordnung zu verhindern, können mit Vorteil an dem Bestätigungshebelarm und/oder an dem Grundkorpus Anschlagmittel zum Begrenzen eines Hebelweges des Betätigungshebelarms in Schließrichtung vorgesehen sein.

Für die Erzeugung einer ausreichend hohen Riegelkraft, die auf das Riegelmittel wirkt, mittels der Hebelanordnung ist es von Vorteil, wenn die Hebelelemente wie in Anspruch 12 angegeben gebildet sind. Demnach sind bei dieser bevorzugten Ausgestaltungsvariante die Hebelelemente jeweils zweiarmige Hebelelemente, wobei das erste Hebelelement als ersten Hebelarm den Betätigungshebelarm und als zweiten Hebelarm einen verglichen mit dem Betätigungshebelarm kürzeren Hebelarm aufweist, der den Lagerverbindungsabschnitt dieses Hebelelementes trägt. Das zweite Hebelelement trägt seinen Lagerverbindungsabschnitt an einem ersten, langen Hebelarm, der länger als der den Lagerverbindungsabschnitt des ersten Hebelelementes tragende Hebelarm ist und hat ferner einen gegenüber seinem ersten Hebelarm kürzeren zweiten Hebelarm, der das Riegelelement bewegt, an dem dieses mit Vorteil direkt angeformt sein kann.

Die Hebelanordnung kann mit Vorteil so gestaltet sein, dass sie einen Kniehebel ausbildet, der bei einer Betätigung zum Bewegen des Riegelmittels in die Riegelposition über einen Totpunkt hinaus bewegbar ist und dort verrastet.

Für die unterschiedlichen Anwendungsformen können an dem Grundkorpus des erfindungsgemäßen Werkzeughalte- und Griffteils Ansatzstrukturen bzw. Einwirkstrukturen für weitere Werkzeuge bzw. Hilfsmittel angeformt sein. So kann z.B. im Bereich des Griffabschnittes ein plattenförmig verdicktes Ende vorgesehen sein als Aufschlagfläche für ein Schlagwerkzeug wie etwa einen Hammer. Auch können Aussparungen oder Vertiefungen vorgesehen sein zum Ansetzen einer meißelartigen Schlaghilfe.

Die Elemente Grundkorpus und Hebelelemente können jeweils einzeln, insbesondere aber insgesamt aus einem biokompatiblen Metall, beispielsweise Edelstahl, gefertigt sein, wobei der Grundkorpus im Bereich des Griffteils mit einer für eine Verbesserung der Griffigkeit und Haptik aufgebrachten Kunststoffummantelung oder einer Abdeckung aus einem anderen Material versehen sein kann, welches mit besonderem Vorteil fest mit dem Basismaterial des Grundkorpus verbunden, z.B. angespritzt ist.

Wie bereits erwähnt ist Bestandteil der Erfindung auch ein medizinisches, ihsbesondere chirurgisches, Instrument, welches aus einem wie vorstehend beschriebenen, erfindungsgemäßen Werkzeughalte- und Griffteil sowie einem mit diesem verbundenen medizinischen, insbesondere chirurgischen, Werkzeug besteht. Das Werkzeug kann dabei insbesondere eine Markraumraspel für das proximale Ende eines menschlichen Femurs sein.

Schließlich ist, wie einstehend bereits ausgeführt, Bestandteil der Erfindung auch ein Set zum Bilden eines medizinischen, insbesondere chirurgischen Instruments wie vorstehend genannt, welches Set ein Werkzeughalte- und Griffteil wie vorstehend beschrieben sowie wenigstens zwei unterschiedliche medizinische, insbesondere chirurgische, Werkzeuge umfasst. Unter unterschiedlichen medizinischen Werkzeugen sind dabei einerseits Werkzeuge gleicher Funktion (beispielsweise Knochenraspeln für das proximale Ende eines menschlichen Femurs) zu verstehen, die unterschiedlich geformt oder dimensioniert sind. Gemeint sind aber auch Werkzeuge unterschiedlicher Funktionalität, beispielsweise Raspeln und Messlehren. Unter den unterschiedlichen medizinischen Werkzeugen können dabei auch Implantatteile sein, die mit einem erfindungsgemäßen Werkzeughalte- und Griffteil erfasst und gesetzt werden können.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: eine teilweise geschnittene schematische Seitenansicht eines Ausführungsbeispiels für ein Werkzeughalte- und Griffteil gemäß der Erfindung ohne angesetztes medizinisches Werkzeug und in zusammengefügtem, Zustand mit dem Riegelmittel in Freigabeposition;
- Fig. 2: eine Ansicht des Werkzeughalte- und Griffteils gemäß Fig. 1 in zerlegtem Zustand mit teilweise geschnittener Darstellung eines Abschnittes des Grundkorpus;
- Fig. 3: eine Darstellung des Werkzeughalte- und Griffteils gemäß Fig. 1 mit in den Grundkorpus eingesetzten Hebelelementen vor einem Verbinden dieser Hebelelemente miteinander bzw. nach einem Lösen derselben voneinander; und
- Fig. 4: in zwei Ansichten a und b ausschnittsweise und teilgeschnittene Darstellungen des Werkzeughalte- und Griffteils gemäß Fig. 1 mit daran angesetztem medizinischen Werkzeug in einer Freigabeposition (Fig. 4a) und in einer Riegelposition (Fig. 4b).

In den Figuren ist in schematischen Skizzen ein mögliches Ausführungsbeispiel eines erfindungsgemäßen Werkzeughalte- und Griffteils für ein medizinisches, insbesondere chirurgisches, Werkzeug dargestellt in unterschiedlichen Ansichten und Zuständen. Die Figuren sind dabei keinesfalls maßstabsgerecht und stellen auch nicht die vollständige und detaillierte Konstruktion eines entsprechenden Ausführungsbeispiels dar. Sie sind vielmehr als Skizzen zu verstehen, die die Grundprinzipien und den wesentlichen Aufbau des Ausführungsbeispiels darstellen, dort detaillierter wiedergeben, wo erfindungsgemäße Merkmale gezeigt und zu erläutern sind.

Nachfolgend wird die Erfindung nun unter Bezugnahme auf die Figuren anhand des dort gezeigten Ausführungsbeispiels näher erläutert und in einer möglichen Ausgestaltungsvariante beschrieben.

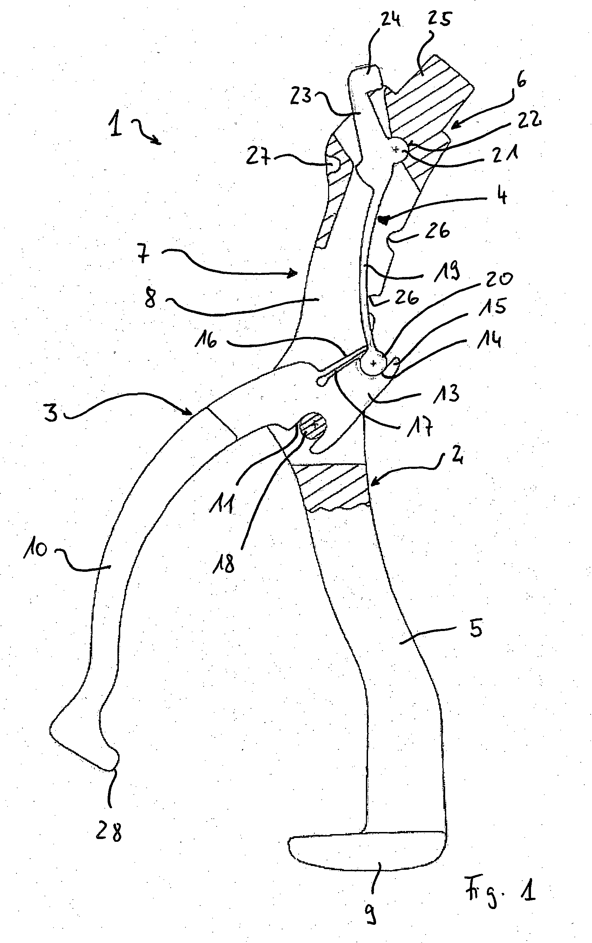

Zunächst wird zur Beschreibung des Ausführungsbeispiels auf Fig. 1 Bezug genommen. Dort ist ein erfindungsgemäß ausgestaltetes Werkzeughalte- und Griffteil für ein medizinisches, insbesondere chirurgisches, Werkzeug allgemein mit der Bezugsziffer 1 bezeichnet. Dieses ist gebildet aus einem Grundkorpus 2 und zwei Hebelelementen 3 und 4, die an dem Grundkorpus 2 verschwenkbar angelagert sind und miteinander gelenkig verbunden sind und so eine Hebelanordnung bilden.

Der Grundkorpus 2 weist einen Griffabschnitt 5 sowie einen Verbindungsabschnitt 6 auf. Griffabschnitt 5 und Verbindungsabschnitt 6 sind einstückig miteinander verbunden, wobei der Grundkorpus 2 wie auch die Hebelelemente 3, 4 insbesondere aus einem metallischen Werkstoff, vorzugsweise aus Edelstahl, gebildet ist. In einem zwischen dem Griffabschnitt 5 und dem Verbindungsabschnitt 6 gelegenen Lagerabschnitt 7 ist in dem Grundkorpus 2 ein Aufnahmeschlitz 8 gebildet, der zumindest teilweise in einer Richtung quer zur Längserstreckungsrichtung des Grundkorpus 2 durchgehend gebildet ist. In der Darstellung der Fig. 1 ist der Grundkorpus 2 teilweise geschnitten dargestellt, wobei der Schnitt sich durch den Aufnahmeabschnitt 6, den Lagerabschnitt 7 bis hin zu einem dem Aufnahmeabschnitt 6 zugewandten Ende des Griffabschnittes 5 erstreckt. Der weitere Griffabschnitt 5 ist ohne einen Längsschnitt dargestellt.

An einem dem Aufnahmeabschnitt 6 gegenüberliegenden Ende des Griffabschnittes 5 befindet sich eine plattenartige Vergrößerung 9, die einerseits eine Begrenzung des Griffabschnittes 5 darstellt und ein Abrutschen einer den Griffabschnitt 2 ergreifenden Hand in Richtung des mit der plattenartigen Vergrößerung 9 versehenen Endes verhindert, andererseits als Aufschlagfläche für das Aufbringen von schlagartigen Stößen beispielsweise mit einem hammerartigen Werkzeug, auf das Werkzeughalte- und Griffteil 1 dient. Der Griffabschnitt 5 des Werkzeughalte- und Griffteils 1 ist in hier nicht näher dargestellter Weise vorzugsweise mit einer die Griffigkeit und Haptik verbessernden Beschichtung oder Ummantelung, insbesondere aus einem Kunststoff oder Gummi, versehen, insbesondere umgossen oder umspritzt.

Die beiden Hebelelemente 3, 4 sind jeweils zweiarmig und ohne weitere Verbindungs- oder Befestigungsmittel unmittelbar und lösbar an entsprechenden Lagerstrukturen des Grundkorpus 2 verschwenkbar gelagert und miteinander zur Ausbildung eines Verbindungsschwenklagers ebenfalls ohne weitere Verbindungsmittel unmittelbar und lösbar verbunden, wie dies nachstehend weiter beschrieben ist.

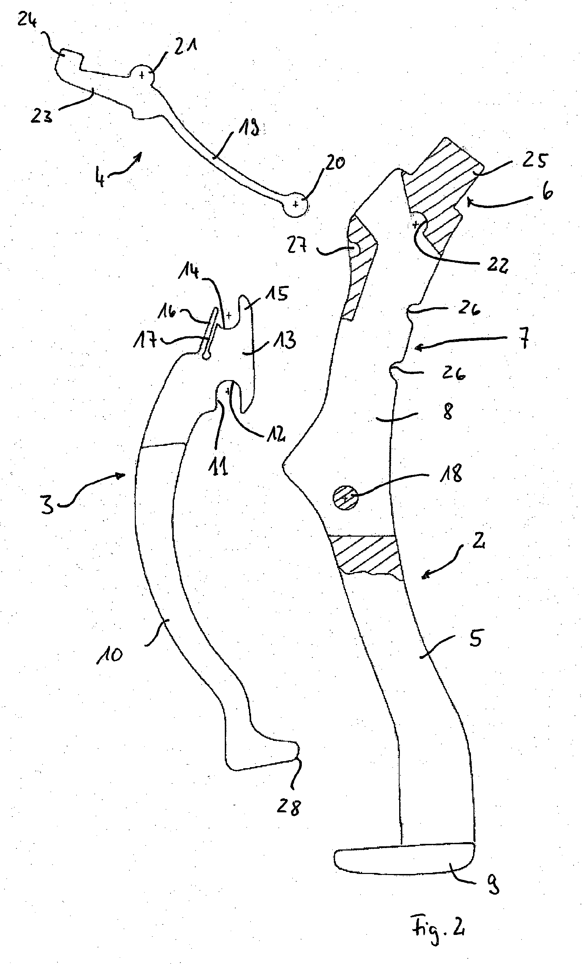

Ein erstes Hebelelement, das Hebelelement 3, umfasst als ersten Hebelarm einen Betätigungshebelarm 10. Dieser Betätigungshebelarm 10 dient der Betätigung der Hebelanordnung durch einen Verwender des Werkzeughalte- und Griffteils 1 in später noch zu beschreibender Weise. Dieser Betätigungshebelarm 10 ist der längere Hebelarm des Hebelelementes 3. Er erstreckt sich bis zu einem Drehpunkt des Hebelelementes 3, welcher durch eine als Lagerstruktur dienende U-förmige Lagerkehle 11 definiert ist. Diese U-förmige Lagerkehle 11 hat einen kreisabschnittförmigen Kehlengrund 12 (vgl. Fig. 2). Ausgehend von der den Drehpunkt des Hebels definierenden Lagerkehle 11 auf der gegenüberliegenden Seite des Betätigungshebelarms 10 ist der zweite Hebelarm 13 des ersten Hebelelementes 3 ausgeformt. Dieser Hebelarm 13 ist verglichen mit dem Betätigungshebelarm 10 ein kürzerer. An seinem der Lagerkehle 11 abgewandten Ende weist der Hebelarm 13 einen Lagerverbindungsabschnitt in Form einer Aufnahmegabel 14 auf. Diese Aufnahmegabel 14 ist ebenso wie die Lagerkehle 11 mit einem am Gabelgrund kreisabschnittsförmig gebildeten Querschnitt versehen. In Richtung senkrecht zur Zeichenebene erstrecken sich die Lagerkehle 11 bzw. die Aufnahmegabel 14 mit gleichmäßigem Querschnitt, so dass der jeweilige im Querschnitt kreisabschnittförmige Grund in dreidimensionaler Ansicht einen Kreiszylinderabschnitt bildet.

Die Aufnahmegabel 14 ist begrenzt durch einen länger geführten feststehenden ersten Schenkel 15 und einen zweiten, kürzer ausgebildeten Schenkel 16. Dieser zweite Schenkel 16 ist aufgrund des diesen vom restlichen Material des Hebelelementes 3 freistellenden Schlitzes 17 als Federzunge ausgebildet mit einer Federelastizität in Richtung quer zu seiner Längserstreckung und in der Zeichenebene der Figur 1.

Mit der Lagerkehle 11 ist das erste Hebelelement 3 auf einen fest in dem Lagerabschnitt 7 integrierten, einen festen Bestandteil des Grundkorpus 2 bildenden im Querschnitt kreisförmigen Lagerstift 18 aufgesetzt. Dieser Lagerstift 18 im Zusammenwirken mit der Lagerkehle 11, insbesondere des Kehlengrundes 12 (vgl. Fig. 2) bildet das Schwenklager des ersten Hebelelementes 3.

Das zweite Hebelelement 4 ist ebenfalls ein zweiarmiges. Ein erster Hebelarm 19 dieses Hebelelementes 4 ist der längere Hebelarm dieses Hebelelementes 4 und zudem länger bemessen als der zweite Hebelarm 13 des ersten Hebelelementes 3. Dieser Hebelarm 19 besitzt an seinem äußeren Ende einen einstückig an diesem angeformten Kreiszylinderabschnitt 20, der einen Lagerverbindungsabschnitt des Hebelelementes 4 bildet. Der Hebelarm 19 ist in seiner Dicke geringer bemessen als der Radius des Kreiszylinderabschnittes 20 und verläuft gekrümmt. Materialstärke, weitere Dimensionen und Materialwahl des Hebelelementes 4 in dessen Hebelarm 19 sind derart bemessen, dass dieser Hebelarm 19 federelastisch quer zu seiner Längserstreckungsrichtung und in der Zeichenebene der Figur 1 verformbar ist, mithin eine Federwirkung entfalten kann.

Das Hebelelement 4 weist einen Lagerabschnitt 21 in Form eines einstückig an diesem Hebelelement 4 angeformten weiteren Kreiszylinderabschnittes auf. Mit diesem Lagerabschnitt 21 sitzt das Hebelelement 4 verschwenkbar gelagert in einer kreiszylinderabschnittförmig, im Querschnitt also kreisförmig im Radius entsprechend gebildeten Aufnahmekehle 22, die in einem dem Verbindungsabschnitt 6 nahegelegenen Abschnitt des Grundkorpus 2 ausgebildet ist. Die Aufnahmekehle 22 erstreckt sich dabei über einen geringeren Umfangsabschnitt als 180°, so dass das Hebelelement 4 mit seinem Lagerabschnitt 21 in die Aufnahmekehle 22 eingesetzt und aus dieser entnommen werden kann. Zusammen definieren Lagerabschnitt 21 und Aufnahmekehle 22 den Drehpunkt des Hebelelementes 4.

Ein zweiter Hebelarm 23 des Hebelelementes 4 erstreckt sich in Richtung einer dem ersten Hebelarm 19 gegenüberliegenden Seite des Lagerabschnittes 21 und ist in seiner Länge kürzer als der erste Hebelarm 19. An seinem äußeren Ende trägt dieser Hebelarm 23 einen im Querschnitt kreisförmigen Riegelzapfen 24, der in später noch zu schildernder Weise dem Verriegeln eines im Verbindungsabschnitt 6 auf das Werkzeughalte- und Griffteil 1 aufgesetzten Werkzeuges dient.

Die beiden Hebelelemente 3 und 4 sind durch eine Verbindung des in die Aufnahmegabel 14 an dem zweiten Arm 13 des ersten Hebelelementes 3 eingeführten, an dem äußeren Ende des ersten Hebelarmes 19 des zweiten Hebelelementes 4 angeordneten Kreiszylinderabschnittes 20 mit dieser Aufnahmegabel 14 miteinander gelenkig verbunden. Dabei ist eine Öffnungsweite der Aufnahmegabel 14, die durch einen Abstand zwischen den freien Enden der Schenkel 15 und 16 bestimmt ist, geringer bemessen als der Durchmesser des Kreiszylinderabschnittes 20. So wird diese Verbindung gesichert. Aufgrund der Federelastizität des als Federzunge ausgebildeten zweiten Schenkels 16 kann der Kreiszylinderabschnitt 20 in die Aufnahmegabel 14 eingedrückt und aus dieser herausgeführt werden, wobei eine Federkraft der Federzunge 16 zu überwinden ist, die bei einem entsprechenden Vorgang abgespreizt wird, beim Einführen des Kreiszylinderabschnittes 20 in die Aufnahmegabel 14 diesen am Ende durch Zurückfedern in ihre Ausgangsposition verrastend dort sichert.

In der wie in Fig. 1 dargestellten zusammengefügten Hebelanordnung, gebildet durch die miteinander gelenkig verbundenen Hebelelemente 3 und 4, bilden diese eine nach dem Kniehebelprinzip arbeitende Anordnung. Denn die Summe der Streckenlänge gerechnet vom Kreismittelpunkt des in der Aufnahmegabel 14 gebildeten kreisförmigen Kehlengrundes bis zum Mittelpunkt des Kreises mit dem Radius des kreisabschnittförmigen Kehlengrundes 12 in der Lagerkehle 11 sowie der Streckenlänge vom Mittelpunkt des Kreiszylinderabschnittes 20 bis zum Mittelpunkt des Kreiszylinderabschnittes am Lagerabschnitt 21 ist größer als die Entfernung des Mittelpunktes des kreisförmigen Querschnittes des Lagerstiftes 18 bis zum Mittelpunkt eines Kreises, der den Radius der im Querschnitt kreisförmigen Aufnahmekehle 22 bestimmt. Zur Kompensation dieser Streckendifferenz ist der Hebelarm 19 wie oben beschrieben federelastisch gebildet. Bei einer Schließbewegung, bei der der Betätigungshebelarm 10 aus der in Fig. 1 dargestellten Stellung in Richtung des Griffabschnittes 5 des Grundkorpus 2 bewegt wird, bewegt die Hebelanordnung sich derart, dass der Hebelarm 23 den Riegelzapfen 24 in Richtung eines im Verbindungsabschnitt 6 ausgebildeten Aufsatzzapfens 25 bewegt. Dabei wird bei dieser Schließbewegung die Hebelanordnung unter Überwindung einer durch die federelastische Verformung des ersten Hebelarms 19 des zweiten Hebelements 4 zunächst gestreckt, und es wird dann ein Totpunkt überschritten, so dass bei in die Riegelposition verschwenktem Riegelzapfen 24 die Hebelanordnung verrastet und gegen ein versehentliches Zurückbewegen in Öffnungsstellung aufgrund der Federkraft des federnden Hebelarmes 19 blockiert ist. Erst eine bewusste, die Federkraft des Hebelarmes 19 überwindende Öffnungsbewegung vermag den Riegelzapfen 24 aus der Riegelstellung zurückzubewegen in die Offenstellung.

In Fig. 1 sind auch noch zwei Ansatzkehlen 26 sowie ein Sackloch 27 zu erkennen, die an dem Grundkorpus 2 ausgebildet sind und dem Ansetzen eines meißelähnlichen Schlagübertragungselementes dienen, um vermittels entsprechender Schläge Kräfte auf das Werkzeughalte- und Griffteil 1 und somit auf ein daran befestigtes Werkzeug übertragen zu können, beispielsweise um dieses im Falle einer Markraumraspel als Werkzeug in den Markraum eines Knochens eintreiben bzw. aus diesem wieder lösen zu können.

Schließlich ist im Bereich des äußeren Ende des Betätigungshebels 10 ein in Richtung des Griffteils 5 weisender Fortsatz 28 zu erkennen, der bei einer Schließbewegung des Betätigungshebels 10 an einer Anschlagfläche des Griffabschnittes 5 anschlägt und somit einen Anschlag zu Begrenzung einer weitergehenden Schließbewegung des Hebelelementes 3 und damit der Hebelanordnung bildet. So kann insbesondere verhindert werden, dass bei einem "Überschließen" die Hebelanordnung an dem Verbindungsschwenklager gelöst wird.

In Fig. 2 ist das erfindungsgemäße Werkzeughalte- und Griffteil 1 aus Fig. 1 in einem in seine drei Einzelteile, nämlich Grundkorpus 2, erstes Hebelelement 3 und zweites Hebelelement 4 zerlegten Zustand gezeigt. Diese drei Teile sind die einzigen Teile, die bei einem Zerlegen des Werkzeughalte- und Griffteils 1 erhalten werden, insbesondere gibt es keine weiteren Verbindungselemente oder sonstigen Kleinteile. Bezüglich der einzelnen, in Fig. 2 ebenfalls mit Bezugszeichen versehenen Teile und Elemente der drei Einzelteile wird hinsichtlich einer entsprechenden Beschreibung Bezug genommen auf die Erläuterungen zur Figur 1, die hier in gleicher Weise zutreffen. Insbesondere nach einem Gebrauch des Werkzeughalte- und Griffteils 1 bspw. im Rahmen einer orthopädischen und/oder chirurgischen Operation kann dieses einfach zerlegt und können die drei Teile (Grundkorpus 2, Hebelement 3, Hebelelement 4) einfach, schnell und zuverlässig gereinigt und sterilisiert werden. Auch lässt sich das Werkzeughalte- und Griffteil 1 anschließend (wie sich aus nachstehenden Erläuterungen noch ergeben wird) wieder einfach für einen nächsten Einsatz durch Zusammenfügen der drei Teile herrichten.

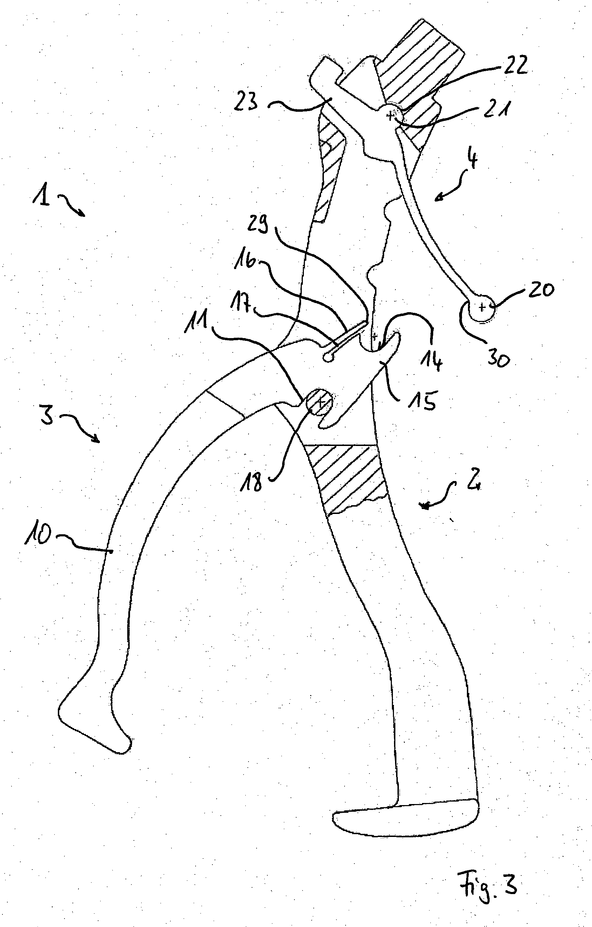

In Fig. 3 ist nun eine - ebenso wie Fig. 1 und Fig. 2 mit teilgeschnittener Darstellung des Grundkorpus 2 wiedergegebene Ansicht des Werkzeughalte- und Griffteils 1 in einem teilweise zusammengesetzten bzw. teilzerlegten Zustand gezeigt. Zu erkennen ist hier, dass die beiden Hebelelemente 3, 4 - im Falle eines Zusammenbaus bereits, im Falle eines Zerlegens noch - zur Ausbildung der jeweiligen Schwenklager an den Grundkorpus 2 angelenkt bzw. in diesen eingesetzt sind. Hierzu ist das erste Hebelelement 3 mit seiner Lagerkehle 11 auf den Lagerstift 18 aufgesetzt. Das zweite Hebelelement 4 ist mit seinem Lagerabschnitt 21 in die Aufnahmekehle 22 an dem Grundkorpus 2 eingesetzt. Da die Dimensionen und Öffnungsweiten der Lagerkehlen 11 bzw. der Aufnahmekehle 22 derart gestaltet sind, dass die Hebelelemente 3 und 4 mit der Lagerkehle 11 bzw. dem Lagerabschnitt 21 einfach über den Lagerstift 18 geführt bzw. in die Aufnahmekehle 22 eingesetzt werden können, kann der in Fig. 3 gezeigte Zustand beim Zusammenbau einfach durch entsprechendes Zusammenbringen und Einführen der Hebelelemente 3 und 4 mit dem Grundkorpus 2 bzw. in diesen erreicht werden. Beim Zerlegen können aus der in Fig. 3 gezeigten Situation heraus die Hebelelemente 3 und 4 einfach von dem Grundkorpus 2 gelöst und entnommen werden.

Zum Erhalten des fertig zusammengebauten Zustandes beim Zusammenbau aus der Situation gemäß Fig. 3 heraus ist nun noch das Verbindungsschwenklager, mit welchem die beiden Hebelelemente 3 und 4 verbunden sind, zu bilden bzw. zu schließen. Dazu können das erste Hebelelement 3 und das zweite Hebelelement 4 derart verschwenkt werden, dass der Kreiszylinderabschnitt 20 zwischen die Enden der Schenkel 15 und 16, die die Aufnahmegabel 14 am ersten Hebelelement 3 begrenzen, positioniert wird. Durch Aufbringen einer Verbindungskraft wird dann der Kreiszylinderabschnitt 20 in die Aufnahmegabel 14 hineingedrückt, wobei der als Federzunge ausgebildete zweite Schenkel 16 zunächst nach außen gedrückt wird und anschließend in seine Ausgangsposition zurückfedert. Auf diese Weise wird - verrastend gehalten - die in Fig. 1 dargestellte Situation erhalten.

Beim Zerlegen des Werkzeughalte- und Griffteils wird die in Fig. 3 dargestellte Position erreicht, indem der Kreiszylinderabschnitt 20 unter Aufbringen einer Lösekraft, die den als Federzunge ausgebildeten Schenkel 16 nach außen drückt, aus der Aufnahmegabel 14 gelöst und so die Verbindung der Hebelelemente 3 und 4 in dem Verbindungsschwenklager gelöst wird. Ein entsprechendes Lösen geschieht dabei durch ein Fortführen der Öffnungsbewegung des Hebelelementes 3, also ein weiteres Abspreizen des Betätigungshebelarms 10 vom Grundkorpus 2. Dabei schlägt das vordere, freie Ende 29 des als Federzunge ausgebildeten Schenkels 16 an einer Übergangskante 30 an, die zwischen dem Kreiszylinderabschnitt 20 und dem weiteren Verlauf des Hebelarms 19 im Bereich des Überganges gebildet ist. Bei einer weiteren Schwenkbewegung des Betätigungshebelarms 10 in Öffnungsrichtung wird dann der Schenkel 16 gegen die durch seine Federwirkung aufgebrachte Rückstellkraft nach auswärts gedrückt, so dass die Öffnungsweite der Aufnahmegabel 14 vergrößert wird und der Kreiszylinderabschnitt 20 aus der Aufnahmegabel 14 gleiten kann und das Verbindungsschwenklager getrennt ist.

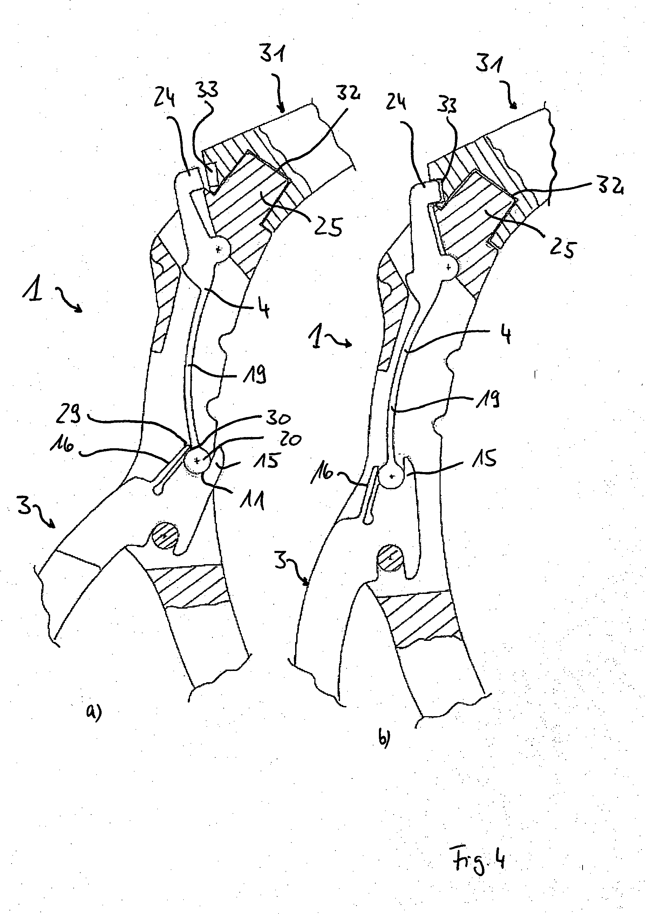

In Fig. 4 ist in zwei Ausschnittsdarstellungen mit unterschiedlicher Stellung des Riegelzapfens 24, einmal in Freigabestellung (Fig. 4a), einmal in Riegelstellung (Fig. 4b) ein Teilausschnitt des Werkzeughalte- und Griffteils 1 dargestellt mit einem Abschnitt eines daran angeordneten medizinischen Werkzeuges 31, welches in einer teilweise geschnittenen Darstellung gezeigt ist. Zu erkennen ist, dass das medizinische Werkzeug 31 mit einer Zapfenaufnahme 32 auf den Aufsatzzapfen 25 aufgesetzt ist. Zu diesem Zwecke ist für eine eindeutige und richtige Orientierung des Werkzeuges 31 der Aufsatzzapfen 25 insbesondere mit einer von der Kreisform abweichenden Querschnittskontur gebildet. Diese ist insbesondere trapezförmig mit aufeinander zu verlaufenden Längskanten sowie gerundeten und kürzeren Querkanten.

Ferner ist an dem medizinischen Werkzeug 31 eine sacklochartige Riegelzapfenaufnahme 33 zu erkennen, in die der Riegelzapfen 24 in der Riegelstellung (Fig. 4b) einfährt und sitzt und das Werkzeug 31 verriegelt, aus der der Riegelzapfen 34 in der Freigabestellung (Fig. 4a) vollständig entfernt ist und eine Entnahme des Werkzeuges 31 von dem Aufsatzzapfen 25 erlaubt.

Zu erkennen ist hier auch noch einmal, wie in der Stellung der Freigabeposition, also im maximal geöffneten Zustand, das vordere Ende 29 des als Federzunge gebildeten Schenkels 16 an der Übergangskante 30 anliegt. Im Falle einer weiteren Öffnungsbewegung des ersten Hebelelementes 3 wird nun geführt durch die Aufnahmegabel 14 und den Schenkel 15 der Kreiszylinderabschnitt 20 aus der Aufnahmegabel 14 herausgedrückt, wobei der als Federzunge ausgebildete Schenkel 16 abgespreizt wird. Auf diese Weise kann eine Trennung des Verbindungsschwenklagers erfolgen zum Lösen der verbundenen Hebelanordnung und für ein nachfolgendes einfaches Zerlegen des Werkzeughalte- und Griffteils 1 durch einfache Entnahme der beiden Hebelelemente 3, 4.

Ebenfalls ist hier deutlich, dass der gegenüber dem Schenkel 16 in der Materialstärke stärker ausgebildete Schenkel 15 in der Aufnahmegabel 14, der ohne elastische Federeigenschaften gebildet ist, derjenige ist, der die Kraft beim Schließen bzw. beim Verbringen des Riegelzapfens 24 in die in Fig. 4b gezeigte Riegelstellung auf das zweite Hebelelement 4 überträgt. Weiterhin ist zu erkennen, dass die Krümmung des ersten Hebelarms 19 des zweiten Hebelelementes 4 der Richtung entgegengesetzt verläuft, in welcher die Schließkraft von dem ersten Hebelelement 3 auf den Hebelarm 19 des zweiten Hebelelementes 4 übertragen wird.

In der in Fig. 4b gezeigten Position, in der das medizinische Werkzeug 31 fest mit dem Werkzeughalte- und Griffteil 1 verbunden ist, ist ein erfindungsgemäßes medizinisches Instrument gebildet. Dabei kann das Werkzeug 31 beispielsweise eine Knochenraspel, insbesondere eine solche für den Markraum des proximalen Femurendes sein. Es kann aber auch ein anderes Werkzeug sein, wobei unter dem Begriff Werkzeug auch Implantatteile oder dgl. zu verstehen sind. Ein erfindungsgemäßes Set zum Bilden eines medizinischen Instrumentes erhält man, wenn wenigstens zwei unterschiedliche medizinische Werkzeuge 31 mit einem Werkzeughalte- und Griffteil 1 kombiniert werden.

### Bezugszeichenliste

- 1: Werkzeughalte- und Griffteil
- 2: Grundkorpus
- 3: Hebelement
- 4: Hebelelement
- 5: Griffabschnitt
- 6: Verbindungsabschnitt
- 7: Lagerabschnitt
- 8: Aufnahmeschlitz
- 9: plattenartige Vergrößerung
- 10: Betätigungshebelarm
- 11: Lagerkehle
- 12: Kehlengrund
- 13: Hebelarm
- 14: Aufnahmegabel
- 15: Schenkel
- 16: Schenkel
- 17: Schlitz
- 18: Lagerstift
- 19: Hebelarm
- 20: Kreiszylinderabschnitt
- 21: Lagerabschnitt
- 22: Aufnahmekehle
- 23: Hebelarm
- 24: Riegelzapfen
- 25: Aufsatzzapfen
- 26: Ansatzkehle
- 27: Sackloch
- 28: Fortsatz
- 29: vorderes Ende
- 30: Übergangskante
- 31: medizinisches Werkzeug
- 32: Zapfenaufnahme
- 33: Riegelzapfenaufnahme

## Patentansprüche

1. Werkzeughalte- und Griffteil zum lösbaren Verbinden mit einem medizinischen, insbesondere chirurgischen, Werkzeug (31), welches einen Grundkorpus (2) mit einem Griffabschnitt (5) und einem Verbindungsabschnitt (6) zum Verbinden mit dem medizinischen Werkzeug (31) aufweist, wobei in dem Verbindungsabschnitt (6) ein aus einer Freigabeposition in eine Riegelposition bewegbares Riegelmittel (24) zum Verriegeln eines an dem Verbindungsabschnitt (6) anzusetzenden medizinischen Werkzeuges (31) vorgesehen ist, wobei das Werkzeughalte- und Griffteil (1) ferner eine Hebelanordnung aus miteinander verbundenen, jeweils an dem Grundkorpus (2) schwenkbar gelagerten Hebelelementen (3, 4) aufweist, von denen ein erstes (3) einen Betätigungshebelarm (10) aufweist, ein zweites (4) das, insbesondere an einem Hebelarm (23) dieses Hebelelementes (4) angeformte, Riegelmittel (24) bewegt, wobei die Hebelanordnung aus zwei Hebelelementen (3, 4) gebildet ist, die jeweils mit daran ausgeformten Schwenklagerabschnitten (11, 12, 21) gegenüber dem Grundkorpus (2) verschwenkbar an in dem Grundkorpus (2) ausgeformten Schwenklagerstrukturen (18, 22) angeordnet sind und jeweils an einem ihrer Hebelarme (13, 19) einen Lagerverbindungsabschnitt 14, 20) aufweisen, welche Lagerverbindungsabschnitte (14, 20) lösbar und unmittelbar miteinander verbunden sind und im Zusammenwirken ein relativ zu dem Grundkorpus (2) verlagerbares Verbindungsschwenklager für die beiden Hebelelemente (3, 4) bilden, **dadurch gekennzeichnet, dass** die beiden Hebelelemente (3, 4) mit ihren Schwenklagerabschnitten (11, 12, 21) an den in dem Grundkorpus (2) ausgeformten Schwenklagerstrukturen (18, 22) unmittelbar und abnehmbar angeordnet sind.

2. Werkzeughalte- und Griffteil nach Anspruch **1**, **dadurch gekennzeichnet, dass** der Lagerverbindungsabschnitt (20) an einem der Hebelelemente (4) einen mit seiner Längsrichtung quer zu der Erstreckungsrichtung des Hebelarmes (19), an dem er angeordnet ist, verlaufenden, an diesem Hebelarm (19) festgelegten, insbesondere einstückig angeformten, Kreiszylinderabschnitt aufweist, und dass der Lagerverbindungsabschnitt (14) an dem anderen der Hebelelemente (3) eine in einem Gabelgrund mit einem zu einem Radius des Kreiszylinderabschnitts korrespondierenden Radius ausgeformte, durch seitliche Schenkel (15, 16) begrenzte Aufnahmegabel umfasst.

3. Werkzeughalte- und Griffteil nach Anspruch 2, **dadurch gekennzeichnet, dass** die Schenkel (15, 16) der Aufnahmegabel (14) ungleich lang sind, wobei der bei einer Schließbewegung der Hebelanordnung, die diese beim Bewegen des Riegelmittels (24) in die Riegelposition ausführt, Kraft übertragende Schenkel (15) der längere ist.

4. Werkzeughalte- und Griffteil nach einem der Ansprüche 2 oder 3, **dadurch gekennzeichnet, dass** die Schenkel (15, 16) der Aufnahmegabel (14) an ihren freien Enden eine Öffnungsweite haben, die geringer ist als der Durchmesser des Kreiszylinderabschnitts (20), und dass einer der Schenkel (16), im Falle des Rückbezuges dieses Anspruchs auf Anspruch 3 insbesondere der kürzere Schenkel (16), als Federzunge ausgebildet ist.

5. Werkzeughalte- und Griffteil nach einem der vorhergehenden Ansprüche, **dadurch gekennzeichnet, dass** einer der Hebelarme (13, 19) der Hebelelemente (3, 4), an denen die Lagerverbindungsabschnitte (14, 20) angeordnet sind, insbesondere der mit dem Lagerverbindungsabschnitt (20) versehene Hebelarm (19) des das Riegelelement (24) bewegenden zweiten Hebelelementes (4), als Federarm ausgebildet ist mit einer Federelastizität in einer Richtung quer zu seiner Längserstreckung.

6. Werkzeughalte- und Griffteil nach Anspruch 5, **dadurch gekennzeichnet, dass** der als Federarm ausgebildete Hebelarm (19) in einer der Kraft, die bei einer Schließbewegung der Hebelanordnung, die diese beim Bewegen des Riegelmittels (24) in die Riegelposition ausführt, auf diesen wirkenden Kraft entgegen gesetzten Richtung gekrümmt verläuft.

7. Werkzeughalte- und Griffteil nach einem der vorhergehenden Ansprüche, **dadurch gekennzeichnet, dass** die in dem Verbindungsschwenklager lösbar verbundenen Schwenklagerabschnitte (14, 20) und die Hebelelemente (3, 4) derart ausgebildet sind, dass bei einem Überstrecken der Hebelanordnung, insbesondere bei einer Öffnungsbewegung derselben, die diese beim Bewegen des Riegelmittels (24) in die Freigabeposition ausführt, die Schwenklagerabschnitte (14, 20) durch Überwinden einer Rückhaltekraft voneinander trenn- und lösbar sind.

8. Werkzeughalte- und Griffteil nach einem der vorhergehenden Ansprüche, **gekennzeichnet durch** einen an wenigstens einem Hebelelement (4) als Lagerabschnitt (21) ausgebildeten Kreiszylinderabschnitt zum schwenkbaren Lagern des Hebelelements (4) in einer die Schwenklagerstruktur (22) bildenden Aufnahmekehle des Grundkorpus (2) mit einem in einem dem Radius des Kreiszylinderabschnitts (21) entsprechenden Radius gekrümmten Kehlengrund.

9. Werkzeughalte- und Griffteil nach einem der vorhergehenden Ansprüche, **gekennzeichnet durch** eine in wenigstens einem der Hebelelemente (3) als Lagerabschnitt (11) ausgebildete U-förmige Lagerkehle mit einem in einem Radius gekrümmt geführten Kehlengrund (12) zum schwenkbaren Lagern des Hebelelements (3) an einem die Schwenklagerstruktur (18) bildenden, fest an dem Grundkorpus angeordneten Lagerstift mit zu dem Radius des Kehlengrundes (12) korrespondierendem Radius.

10. Werkzeughalte- und Griffteil nach einem der vorhergehenden Ansprüche, **gekennzeichnet durch** einen in einem Abschnitt des Grundkorpus (2) gebildeten, zumindest teilweise durchgehenden Aufnahmeschlitz (8) zum wenigstens teilweise Aufnehmen der Hebelelemente (3, 4), wobei die Schwenklagerstrukturen (18, 22) zum schwenkbaren Lagern der Hebelelemente (3, 4) in dem Aufnahmeschlitz (8) angeordnet sind.

11. Werkzeughalte- und Griffteil nach einem der vorhergehenden Ansprüche, **gekennzeichnet durch** an dem Betätigungshebelarm (10) und/oder an dem Grundkorpus (2) angeordnete Anschlagmittel (28) zum Begrenzen eines Hebelweges des Betätigungshebelarms (10) in einer Schließrichtung, in der das Riegelmittel (24) in die Riegelposition bewegt wird.

12. Werkzeughalte- und Griffteil nach einem der vorhergehenden Ansprüche, **dadurch gekennzeichnet, dass** die Hebelemente (3, 4) jeweils zweiarmige Hebelemente (3, 4) sind, wobei das erste Hebelemente (3) als ersten Hebelarm den einen Betätigungshebelarm (10) und als zweiten Hebelarm einen verglichen mit dem Betätigungshebelarm (10) kürzeren Hebelarm (13), der den Lagerverbindungsabschnitt (14) dieses Hebelementes (3) trägt aufweist, und wobei das zweite Hebelement (4) einen den Lagerverbindungsabschnitt (20) dieses Hebelelementes (4) tragenden langen ersten Hebelarm (19), der länger als der den Lagerverbindungsabschnitt (14) des ersten Hebelelementes (3) tragende Hebelarm (13) ist, und einen gegenüber seinem ersten Hebelarm (19) kürzeren zweiten Hebelarm (23) aufweist, der das Riegelmittel (24) bewegt.

13. Werkzeughalte- und Griffteil nach einem der vorhergehenden Ansprüche, **dadurch gekennzeichnet, dass** die Hebelanordnung einen Kniehebel ausbildet, der bei einer Betätigung zum Bewegen des Riegelmittels (24) in die Riegelposition über einen Totpunkt hinaus bewegbar ist und dort verrastet.

14. Medizinisches, insbesondere chirurgisches, Instrument, bestehend aus einem Werkzeughalte- und Griffteil (1) nach einem der vorstehenden Ansprüche und einem mit diesem verbundenen medizinischen, insbesondere chirurgischen, Werkzeug (31), insbesondere einer Markraumraspel für das proximale Ende eines menschlichen Femurs.

15. Set zum Bilden eines medizinischen, insbesondere chirurgischen, Instruments nach Anspruch 14, bestehend aus einem Werkzeughalte- und Griffteil (1) nach einem der Ansprüche 1 bis 13 sowie wenigstens zwei unterschiedlichen medizinischen, insbesondere chirurgischen, Werkzeugen (31).

## Claims

1. Tool holding and gripping part for releasable connection to a medical, in particular surgical, tool (31), having a main body (2) with a grip portion (5) and a connection portion (6) for connecting to the medical tool (31), wherein a latch mechanism (24), which is movable from a release position to a latching position and which is used for latching a medical tool (31) to be applied to the connection portion (6), is provided in the connection portion (6), wherein the tool holding and gripping part (1) also has a lever arrangement composed of interconnected lever elements (3, 4) which are each mounted pivotably on the main body (2), of which a first (3) has an actuation lever arm (10), and a second (4) moves the latch mechanism (24), which is in particular formed integrally on a lever arm (23) of this lever element (4), wherein the lever arrangement is formed by two lever elements (3, 4) which, with pivot bearing portions (11, 12, 21) formed thereon, are each arranged pivotably in relation to the main body (2) on pivot bearing structures (18, 22) formed in the main body (2) and each have a bearing connection portion (14, 20) on one of their lever arms (13, 19), which bearing connection portions (14, 20) are connected releasably and directly to each other and, in cooperation, form a connection pivot bearing for the two lever elements (3, 4) that is displaceable relative to the main body (2), **characterized in that** the two lever elements (3, 4) with their pivot bearing portions (11, 12, 21) are arranged directly and removably on the pivot bearing structures (18, 22) formed in the main body (2).

2. Tool holding and gripping part according to Claim 1, **characterized in that** the bearing connection portion (20) on one of the lever elements (4) has a circular cylinder portion which has its longitudinal direction extending transversely with respect to the direction of extent of the lever arm (19) on which it is arranged, and which is secured, in particular formed integrally, on this lever arm (19), and **in that** the bearing connection portion (14) on the other of the lever elements (3) comprises a receiving fork which is limited by lateral branches (15, 16) and is formed in a fork bottom with a radius corresponding to a radius of the circular cylinder portion.

3. Tool holding and gripping part according to Claim 2, **characterized in that** the branches (15, 16) of the receiving fork (14) are of different lengths, wherein the branch (15) transmitting force during a closing movement of the lever arrangement, which the latter executes during the movement of the latch mechanism (24) to the latching position, is the longer one.

4. Tool holding and gripping part according to either of Claims 2 and 3, **characterized in that** the branches (15, 16) of the receiving fork (14) have, at their free ends, an opening width that is smaller than the diameter of the circular cylinder portion (20), and **in that** one of the branches (16), in the case of back-reference of this claim to Claim 3 in particular the shorter branch (16), is designed as a resilient tongue.

5. Tool holding and gripping part according to one of the preceding claims, **characterized in that** one of the lever arms (13, 19) of the lever elements (3, 4) on which the bearing connection portions (14, 20) are arranged, in particular the lever arm (19) provided with the bearing connection portion (20) and forming part of the second lever element (4) that moves the latch element (24), is designed as a resilient arm with a spring elasticity in a direction transverse to its longitudinal extent.

6. Tool holding and gripping part according to Claim 5, **characterized in that** the lever arm (19) designed as a resilient arm extends in a curve in a direction counter to the force which is exerted on the latter during a closing movement of the lever arrangement which said lever arrangement executes during the movement of the latch mechanism (24) to the latching position.

7. Tool holding and gripping part according to one of the preceding claims, **characterized in that** the pivot bearing portions (14, 20), connected releasably in the connection pivot bearing, and the lever elements (3, 4) are designed in such a way that, upon a hyperextension of the lever arrangement, in particular during an opening movement that this executes during the movement of the latch mechanism (24) to the release position, the pivot bearing portions (14, 20) are separable and releasable from each other by overcoming a retaining force.

8. Tool holding and gripping part according to one of the preceding claims, **characterized by** a circular cylinder portion formed on at least one lever element (4) as bearing portion (21) for pivotable bearing of the lever element (4) in a receiving throat of the main body (2) forming the pivot bearing structure (22), having a throat bottom curved in a radius corresponding to the radius of the circular cylinder portion (21).

9. Tool holding and gripping part according to one of the preceding claims, **characterized by** a U-shaped bearing throat formed in at least one of the lever elements (3) as bearing portion (11), with a throat bottom (12) curved in a radius for the pivotable bearing of the lever element (3) on a bearing pin forming the pivot bearing structure (18), arranged fixedly on the main body, with a radius corresponding to the radius of the throat bottom (12).

10. Tool holding and gripping part according to one of the preceding claims, **characterized by** a receiving slit (8), formed at least partially continuously in a portion of the main body (2), for at least partially receiving the lever elements (3, 4), wherein the pivot bearing structures (18, 22) for the pivotable bearing of the lever elements (3, 4) are arranged in the receiving slit (8).

11. Tool holding and gripping part according to one of the preceding claims, **characterized by** abutment means (28) which are arranged on the actuation lever arm (10) and/or on the main body (2) and for limiting a lever path of the actuation lever arm (10) in a closing direction in which the latch mechanism (24) is moved to the latching position.

12. Tool holding and gripping part according to one of the preceding claims, **characterized in that** the lever elements (3, 4) are each two-armed lever elements (3, 4), wherein the first lever element (3) has, as first lever arm, the one actuation lever arm (10) and, as second lever arm, a lever arm (13) which is shorter compared to the actuation lever arm (10) and which carries the bearing connection portion (14) of this lever element (3), and wherein the second lever element (4) has a long first lever arm (19), which carries the bearing connection portion (20) of this lever element (4) and is longer than the lever arm (13) carrying the bearing connection portion (14) of the first lever element (3), and has a second lever arm (23) which is shorter in relation to its first lever arm (19) and which moves the latch mechanism (24).

13. Tool holding and gripping part according to one of the preceding claims, **characterized in that** the lever arrangement forms a toggle mechanism which, upon an actuation for moving the latch mechanism (24) to the latching position, is movable beyond a dead point and locks there.

14. Medical, in particular surgical, instrument, composed of a tool holding and gripping part (1) according to one of the preceding claims and of a medical, in particular surgical, tool (31) connected thereto, in particular a medullary rasp for the proximal end of a human femur.

15. Kit for forming a medical, in particular surgical, instrument according to Claim 14, composed of a tool holding and gripping part (1) according to one of Claims 1 to 13 and of at least two different medical, in particular surgical, tools (31).

## Revendications

1. Pièce de maintien et de préhension d'outil destinée à être assemblée de manière amovible avec un outil médical (31), notamment chirurgical, qui présente un corps de base (2) muni d'une portion de manche (5) et d'une portion d'assemblage (6) pour l'assemblage avec l'outil médical (31), un moyen de verrouillage (24) destiné à verrouiller un outil médical (31) à fixer à la portion d'assemblage (6) et pouvant être déplacé d'une position de libération en une position de verrouillage étant prévu dans la portion d'assemblage (6), la pièce de maintien et de préhension d'outil (1) présentant en outre un arrangement de levier composé d'éléments leviers (3, 4) reliés entre eux, respectivement montés de manière pivotante sur le corps de base (2), dont un premier (3) présente un bras de levier d'actionnement (10), un deuxième (4) déplace le moyen de verrouillage (24), notamment façonné sur un bras de levier (23) de cet élément levier (4), l'arrangement de levier étant composé de deux éléments leviers (3, 4) qui sont respectivement disposés de manière à pouvoir pivoter par rapport au corps de base (2), avec des portions de palier de pivotement (11, 12, 21) façonnées sur ceux-ci, sur des structures de palier de pivotement (18, 22) façonnées dans le corps de base (2) et présentent respectivement sur l'un de leurs bras de levier (13, 19) une portion d'assemblage de palier (14, 20), lesquelles portions d'assemblage de palier (14, 20) sont assemblées entre elles de manière amovible et directement et, en interaction, forment pour les deux éléments leviers (3, 4) un palier de pivotement d'assemblage qui peut être décalé par rapport au corps de base (2), **caractérisée en ce que** les deux éléments leviers (3, 4) sont disposés avec leurs portions de palier de pivotement (11, 12, 21) directement et de manière amovible sur les structures de palier de pivotement (18, 22) façonnées dans le corps de base (2).

2. Pièce de maintien et de préhension d'outil selon la revendication 1, **caractérisée en ce que** la portion d'assemblage de palier (20) présente sur l'un des éléments leviers (4) une portion cylindrique circulaire qui s'étend avec son sens longitudinal transversalement au sens de la projection du bras de levier (19) sur lequel elle est disposée, fixée à ce bras de levier (19), notamment façonnée d'une seule pièce, et **en ce que** la portion d'assemblage de palier (14) comprend, sur l'autre des éléments leviers (3), une fourche d'accueil façonnée dans une base de fourche avec un rayon correspondant à un rayon de la portion cylindrique circulaire et délimitée par des branches latérales (15, 16).

3. Pièce de maintien et de préhension d'outil selon la revendication 2, **caractérisée en ce que** les branches (15, 16) de la fourche d'accueil (14) sont de longueurs différentes, la branche (15) qui transmet la force lors d'un mouvement de fermeture de l'arrangement de levier, qu'il exécute lors du déplacement du moyen de verrouillage (24) dans la position de verrouillage, étant la plus longue.

4. Pièce de maintien et de préhension d'outil selon l'une des revendications 2 ou 3, **caractérisée en ce que** les branches (15, 16) de la fourche d'accueil (14) présentent à leurs extrémités libres une largeur d'ouverture qui est inférieure au diamètre de la portion cylindrique circulaire (20), et **en ce que** l'une des branches (16), notamment la branche la plus courte (16) dans le cas d'un renvoi de la présente revendication à la revendication 3, est réalisée sous la forme d'une languette flexible.

5. Pièce de maintien et de préhension d'outil selon l'une des revendications précédentes, **caractérisée en ce que** l'un des bras de levier (13, 19) des éléments leviers (3, 4) sur lesquels sont disposées les portions d'assemblage de palier (14, 20), notamment le bras de levier (19) muni de la portion d'assemblage de palier (20) du deuxième élément levier (4) qui déplace l'élément de verrouillage (24), est réalisé sous la forme d'un bras flexible avec une élasticité flexible dans une direction transversale à sa projection longitudinale.

6. Pièce de maintien et de préhension d'outil selon la revendication 5, **caractérisée en ce que** le bras de levier (19) réalisé sous la forme d'un bras flexible s'étend de manière curviligne dans une direction opposée à la force qui est exercée sur celui-ci lors d'un mouvement de fermeture de l'arrangement de levier que celui-ci exécute lors du déplacement du moyen de verrouillage (24) dans la position de verrouillage.

7. Pièce de maintien et de préhension d'outil selon l'une des revendications précédentes, **caractérisée en ce que** les portions de palier de pivotement (14, 20) assemblées de manière amovible dans le palier de pivotement d'assemblage et les éléments leviers (3, 4) sont configurés de telle sorte que lors d'un allongement excessif de l'arrangement de levier, notamment lors d'un mouvement d'ouverture de celui-ci, que celui-ci exécute lors du déplacement du moyen de verrouillage (24) dans la position de libération, les portions de palier de pivotement (14, 20) peuvent être séparées et détachées l'une de l'autre en surmontant une force de retenue.

8. Pièce de maintien et de préhension d'outil selon l'une des revendications précédentes, **caractérisée par** une portion cylindrique circulaire réalisée sur au moins un élément levier (4) sous la forme d'une portion de palier (21) pour supporter en pivotement l'élément levier (4) dans une rainure d'accueil du corps de base (2) formant la structure de palier de pivotement (22) et ayant un fond de rainure incurvé avec un rayon correspondant au rayon de la portion cylindrique circulaire (21).

9. Pièce de maintien et de préhension d'outil selon l'une des revendications précédentes, **caractérisée par** une rainure support en forme de U réalisée en tant que portion de palier (11) dans au moins l'un des éléments leviers (3), comprenant un fond de rainure (12) amené de façon incurvée dans un rayon pour supporter en pivotement l'élément levier (3) sur une broche support formant la structure de palier de pivotement (18), montée à demeure sur le corps de base, avec un rayon correspondant au rayon du fond de rainure (12).

10. Pièce de maintien et de préhension d'outil selon l'une des revendications précédentes, **caractérisée par** une fente d'accueil (8) au moins partiellement continue, formée dans une portion du corps de base (2), pour accueillir au moins partiellement les éléments leviers (3, 4), les structures de palier de pivotement (18, 22) étant disposées dans la fente d'accueil (8) afin de supporter en pivotement les éléments leviers (3, 4).

11. Pièce de maintien et de préhension d'outil selon l'une des revendications précédentes, **caractérisée par** des moyens de butée (28) disposées sur le bras de levier d'actionnement (10) et/ou sur le corps de base (2) pour limiter une course de levier du bras de levier d'actionnement (10) dans un sens de fermeture, dans lequel le moyen de verrouillage (24) est déplacé dans la position de verrouillage.

12. Pièce de maintien et de préhension d'outil selon l'une des revendications précédentes, **caractérisée en ce que** les éléments leviers (3, 4) sont respectivement des éléments leviers (3, 4) à deux bras, le premier élément levier (3) présentant en tant que premier bras de levier l'un des bras de levier d'actionnement (10) et en tant que deuxième bras de levier un bras de levier (13) plus court en comparaison avec le bras de levier d'actionnement (10), lequel porte la portion d'assemblage de palier (14) de cet élément levier (3), et le deuxième élément levier (4) étant un premier bras de levier (19) long qui porte la portion d'assemblage de palier (20) de cet élément levier (4), lequel est plus long que le bras de levier (13) portant la portion d'assemblage de palier (14) du premier élément levier (3), et présente un deuxième bras de levier (23) plus court par rapport à son premier bras de levier (19), lequel déplace le moyen de verrouillage (24).

13. Pièce de maintien et de préhension d'outil selon l'une des revendications précédentes, **caractérisée en ce que** l'arrangement de levier forme un levier à genouillère qui, lors d'un actionnement destiné à déplacer le moyen de verrouillage (24) dans la position de verrouillage, peut être déplacé au-delà d'un point mort et s'y enclenche.

14. Instrument médical, notamment chirurgical, composé d'une pièce de maintien et de préhension d'outil (1) selon l'une des revendications précédentes et d'un outil médical (31), notamment chirurgical, assemblé avec celle-ci, notamment une râpe à espace médullaire pour l'extrémité proximale d'un fémur humain.

15. Ensemble pour former un instrument médical, notamment chirurgical, selon la revendication 14, composé d'une pièce de maintien et de préhension d'outil (1) selon l'une des revendications 1 à 13 ainsi que d'au moins deux outils médicaux (31), notamment chirurgicaux, différents.
